# EUROPEAN PATENT SPECIFICATION

(11) **EP 0 390 596 B1**
(45) Date of publication and mention of the grant of the patent: **16.02.1994**
(21) Application number: 90303451.0
(22) Date of filing: 30.03.1990
(51) Int. Cl.: C07C 2/66, C07C 15/02, C07C 43/04, C07C 41/06

(54) **Combined etherification and alkylation process**
Kombiniertes Verätherungs- und Alkylierungsverfahren
Procédé combiné d'étherification et d'alcoylation

(30) Priority: 30.03.1989 US 330779
(43) Date of publication of application: 03.10.1990
(73) Proprietor: CHEMICAL RESEARCH & LICENSING COMPANY, Pasadena, Texas 77507 (US)
(72) Inventor: Jones, Edward M., Jr., Friendswood, Texas 77546 (US)
(74) Representative: Cropp, John Anthony David

(56) References cited:
- EP-A- 0 179 456
- DE-B- 1 277 230
- US-A- 4 336 407
- US-A- 4 849 569

## Description

The present invention relates to a combined process for the preparation of methyl tertiary butyl ether and butyl benzene. More particularly the invention relates to a process which utilizes the etherification of the isobutene in a mixed butene stream, which preferentially reacts with methanol to form methyl tertiary butyl ether, as a separation step to produce high purity n-butene. The n-butene is then used to alkylate benzene to produce butyl benzene. Both the etherification and alkylation steps are carried out in distillation column reactors in which the distillation structure comprises the catalyst.

### Related Art:

Recently a new method of carrying out catalytic reactions has been developed, wherein the components of the reaction system are concurrently separable by distillation, using the catalyst structures as the distillation structures. Such systems are described variously in U.S. Patents 4,215,011; 4,232,530; 4:242,530; 4,250,052; 4,302,356; 4;307;254 and 4,336,407 commonly assigned herewith.

Particularly patents 4,215,011 and 4,336,407 deal with separation of iso and normal olefins. The first discloses that iso-butene can be separated by feeding a stream of mixed C₄ olefins to a catalytic distillation column containing an acid cation exchange resin packing where the n-butene reacts with itself to form a diisobutene which is concurrently separated from the iso-butene in the distillation column reactor. Similarly, the second discloses that when the mixed C₄ olefin is fed to a similar distillation column reactor containing the same or similar acid cation exchange resin along with methanol the iso-butene preferentially reacts with the methanol to form methyl tertiary butyl ether which is distilled off in the distillation column reactor.

It has also been recently discovered that the distillation column reactor, when packed with a suitable catalyst, is useful for the alkylation of organic aromatic compounds. See for example US-A-4 849 569.

### SUMMARY OF THE INVENTION

Briefly, the present invention is a process for separating iso-butene from n-butene, while at same time producing methyl tertiary butyl ether, and then using the n-butene to alkylate benzene to produce butyl benzene. The separation of the n-butene from iso-butene is achieved by
(a) feeding the mixture of isobutene and n-butene with methanol to a distillation column reactor into a first feed zone,
(b) concurrently in the distillation column reactor
   (1) contacting the mixture and methanol with a fixed bed acid cation exchange resin packing in a distillation reaction zone causing the isobutene to preferentially react with the methanol to form methyl tertiary butyl ether and
   (2) Fractionating the resulting mixture and n-butene in the fixed bed acid cation exchange resin packing,
(c) withdrawing the ether from the distillation column reactor below the first zone and
(d) withdrawing the n-butene from the distillation column reactor above the feed zone.

The resulting high purity n-butene stream is used to alkylate benzene by
(e) feeding the n-butene along with benzene to a second distillation column reactor into a second feed zone,
(f) concurrently in the second distillation column reactor
   (1) contacting the n-butene and the benzene with a second fixed bed acidic catalytic distillation structure, such as the acid cation exchange resin or and acidic molecular sieve, causing the n-butene to react with a portion of the benzene to form butyl benzene and
   (2) fractionating the resultant mixture of unreacted benzene and butyl benzene in the second fixed bed acidic catalytic distillation structure,
(g) withdrawing the butyl benzene from the second distillation column reactor at a point below the second feed zone, and
(h) withdrawing said unreacted benzene from the second distillation column reactor at a point above the second feed zone.

Other embodiments include condensing a portion of the unreacted benzene and returning the condensed benzene to the distillation column reactor to control the molar ratio of benzene to n-butene in the distillation reaction zone.

More specifically the mole sieve or cation exchange resin catalyst packing is of such a nature as to allow vapor flow through the bed, yet provide a sufficient surface area for catalytic contact as described in the previously noted US-A-4 849 569 and U.S. Patent No's 4,215,011 and, 4,302,356 which are incorporated herein in their entirety. The catalyst packing is preferably arranged in the upper portion of the distillation column reactor, more preferably occupying about one-third to one half of the column and extending substantially to the upper end thereof.

In the etherification-separation step the butenes and methanol may be fed to the distillation column reactor together, and preferably the feed zone is into the lower end of the catalyst bed. In the alkylation step the n-butene and benzene are preferably fed separately, with the n-butene being fed to the second distillation column reactor from below to near the middle of the catalyst bed with the benzene being fed near the top of the catalyst bed. For simplicity, the make up benzene may be simply added to the overhead receiver where it is returned to the column along with the reflux.

Conditions used are those that react substantially all of the iso-butene with the methanol in the first distillation column reactor. Additionally the molar ratio of the benzene to n-butene in the second distillation reaction zone is controlled such that the n-butene will not react with itself to produce codimers of the butene as disclosed in U.S. patent 4,336,407 but rather preferentially react with the benzene to form butyl benzene. This is achieved by having a large molar excess of benzene to isobutene in the reaction zone.

### DETAILED DESCRIPTION OF THE PREFERRED EMBODIMENT

The reaction system can be described as heterogeneous since the catalyst remains as a distinct entity. The catalyst may be employed in such conventional distillation packing shapes, as Raschig rings, Pall rings, saddles or the like.

In the MTBE reaction methanol may be and is preferably present in a stoichiometric amount although an excess of up to 10 %, may be desirable. In addition, slightly less than a stoichiometric amount may be employed.

The etherification reaction of isobutene and the fractionation of the resultant n-butene-ether mixture is carried out simultaneously, i.e., concurrently. That is, as the ether is formed in the catalyst bed the lower boiling n-butene is fractionated away in the catalyst bed and removed overhead while the high boiling ether drops to the lower portion of the column.

Mixed C₄ streams containing principally isobutane (I-C₄), normal butane (n-C₄), butene-1 (B-1), isobutene (I-B), trans butene-2 (TB)-2) and cis butene-2 (CB-2) (plus some minor impurities including butadiene), can be treated with cold sulfuric acid to remove isobutene and produce a butylene concentrate. The isobutene removed is recovered from the acid as a polymer (mostly dimer). The isobutene dimer (i.e., some other isobutene polymers as well) and butene concentrate are valuable products.

It has been found that a distillation column packed with a properly supported acid catalyst into which the mixed C₄ stream and methanol are fed can produce a bottom stream containing methyl tertiary butyl ether and an overhead stream that is relatively free of isobutene.

The success of the catalytic distillation approach lies in an understanding of the principles associated with distillation. First, because the reaction is occurring concurrently with distillation, the initial reaction product, e.g., methyl tertiary butyl ether (or diisobutylene) is removed from the reaction zone nearly as quickly as it is formed. This removal of the ether minimizes decomposition of the ether and chaining to form isobutene polymer. Second, because all the C₄ components are boiling, the temperature of the reaction is controlled by the boiling point of the C₄ mixture at the system pressure. The heat of reaction simply creates more boil up, but no increase in temperature. Third, the reaction has an increased driving force because the reaction products have been removed and can not contribute to a reverse reaction (LeChatelier's Principle).

As a result, a great deal of control over the rate of reaction and distribution of products can be achieved by regulating the system pressure. Also, adjusting the through-put (residence time = liquid hourly space velocity ⁻¹) gives further control of product distribution and degree of isobutene removal.

The temperature in the reactor is determined by the boiling point of the C₄'s at any given pressure, that is, at constant pressure a change in the temperature of the system indicates a change in the composition in the column. Thus, to change the temperature the pressure is changed. By increasing the pressure, the temperature in the system is increased. Generally, pressures in the range of 0 to 27,58 bars (0 to 400 psig) are or may be employed, preferably 2,06 to 10,34 bars (30 to 150 psig). For the C₄ stream, the present reaction will be carried out generally at pressures in the range of 0,68 to 20,68 bars (10 to 300 psig), which will generally mean temperatures in the range of 10 to 100°C.

The reaction of isobutene with methanol is equilibrium limited; however, by carrying out the reaction in a distillation column reactor and fractionating the formed product, methyl tertiary butyl ether (MTBE), downward away from the reaction zone, the equilibrium is constantly disrupted and hence the reaction never comes to equilibrium. This has the advantage, of course, of achieving an effective 100% conversion, provided the catalyst bed is of sufficient length such that none of the isobutene escapes therefrom to go overhead with the n-butenes. The adjustment of the size of the catalyst bed is a mere mechanical step to be determined for each reactor and in accordance with the reaction conditions.

The MTBE system would normally be considered anhydrous; however, small amounts of water often saturate the feed stream and represent about 400 to 600 ppm thereof. The process will continue to operate in the same fashion, in the presence of this amount of water. Generally the system will be employed with less than 1 mole % water in the feed. However, the limitation on water is relevant to the MTBE reaction. Quite obviously, where water is a reactant or a principal component of a feed stream according to the generic invention, it may be present in substantially larger amounts as required.

The feed of the distillation column reactor is made at the lower end of the catalyst bed for the MTBE reaction, preferably into the catalyst to allow immediate contact of the isobutene and methanol with the catalyst which is characterized as the feed zone.

A reflux is preferably included in the system. The reflux ratio could vary over the rate of 1 to 20:1. In practice, the higher ratio may be used to compensate for a short catalyst bed such as required for experimental work. In commercial size units the catalyst bed would be provided so that lower reflux and hence higher unit productivity could be obtained.

The present alkylation reaction can be carried out at sub-through super atmospheric pressure, e.g., 0,20 to 40,5 bars (0.20 to 40 atmospheres). Furthermore, the temperature along the column will be as in any distillation column, the highest temperature will be in the bottom and the temperature along the column will be the boiling point of the compositions at that point in the column under the particular conditions of pressure. Moreover, the exothermic heat of reaction does not change the temperature in the column, but merely causes more boil up. However, the temperatures within the column with the above considerations in mind will generally be in the range of 50°C to 500°C, preferably 70°C to 500°C for the mole sieve and 70°C to 200°C for the cation exchange resin, and more preferably in the range of about 80°C to 300°C at pressures of 0,5 to 20,25 bars (0.5 to 20 atmospheres) for the mole sieve, and about 80°C to 150°C at 0,25 to 15,19 bars (.25 to 15 atmospheres) for the resin catalyst.

The mole ratio of organic aromatic compound to olefin may be in the range of 2 to 100 : 1, preferably 2 to 50 : 1 and more desirably about 2 to 10 : 1. The greater the excess of organic aromatic compound the more the selectivity to the monosubstituted product is improved. Alkylation is forced to completion, since the simultaneous and concurrent fractionation and removal of the alkylation product from the distillation column reactor does not allow the products to contribute to the reverse reaction (Le Chatelier's Principle). However, very large molar excesses of organic aromatic compounds require a very high reflux ratio, and a low unit productivity.

A catalysts suitable for either the etherification step or the alkylation step are cation exchange resins which include those which contain sulfonic acid groups, and which may be obtained by polymerization or copolymerization of aromatic vinyl compounds followed by sulfonation. Examples of aromatic vinyl compounds suitable for preparing polymers or copolymers are: styrene, vinyl toluene, vinyl naphthalene, vinyl ethylbenzene, methyl styrene, vinyl chlorobenzene and vinyl xylene. A large variety of methods may be used for preparing these polymers; for example, polymerization alone or in admixture with other monovinyl compounds, or by crosslinking with polyvinyl compounds; for example, with divinyl benzene, divinyl toluene, divinylphenylether and others. The polymers may be prepared in the presence or absence of solvents or dispersing agents, and various polymerization initiators may be used, e.g., inorganic or organic peroxides, persulfates, etc.

The sulfonic acid group may be introduced into these vinyl aromatic polymers by various known methods; for example, by sulfating the polymers with concentrated sulfuric and chlorosulfonic acid, or by copolymerizing aromatic compounds which contain sulfonic acid groups (see e.g., US Pat. No. 2,366,007). Further sulfonic acid groups may be introduced into the polymer which already contain sulfonic acid groups; for example, by treatment with fuming sulfuric acid, i.e., sulfuric acid which contains sulfur trioxide. The treatment with fuming sulfuric acid is preferably carried out at 0 to 150°C and the sulfuric acid should contain sufficient sulfur trioxide so that it still contains 10 to 50% free sulfur trioxide after the reaction. The resulting products preferably contain an average of 1.3 to 1.8 sulfonic acid groups per aromatic nucleus. Particularly suitable polymers which contain sulfonic acid groups are copolymers of aromatic monovinyl compounds with aromatic polyvinyl compounds, particularly divinyl compounds, in which the polyvinyl benzene content is preferably 1 to 20% by weight of the copolymer (see, for example, German Patent Specification 908,240). The ion exchange resin is generally used in a granular size of about 0.25 to 1 mm, although particles from 0.15 mm up to about 2 mm may be employed. The finer catalysts provide high surface area, but also result in high pressure drops through the reactor, The macroreticular form of these catalysts have much larger surface area exposed and limited swelling which all of these resins undergo in a non-aqueous hydrocarbon medium compared to the gelular catalysts.

Molecular sieves which are also suitable for the alkylation step are porous crystalline, three-dimensional alumina-silicates of the zeolite mineral group. The crystal skeleton is composed of silicon and aluminum atoms each surrounded by four oxygen atoms to form a matrix. The term molecular sieve can be applied to both naturally occurring zeolites and synthetic zeolites. Naturally occurring zeolites have irregular pore size and are not generally considered as equivalent to synthetic zeolites. In the present invention, however, naturally occurring zeolites are acceptable so long as they are substantially pure. The balance of the present discussion shall be directed to the synthetic zeolites with the understanding that natural zeolites are considered equivalent thereto as indicated above, i.e. in so far as the natural zeolites are the functional equivalents to the synthetic zeolites.

Usually synthetic zeolites are prepared in the sodium form, that is, with a sodium cation in close proximity to each aluminum tetrahedron and balancing its charge. To date seven principal types of molecular sieves have been reported, A, X, Y, L, erionite, omega and mordenite. The A type have relative small pore sizes. By the term pore size is meant the effective pore size (diameter) rather than the free pore size (diameter). Types X and Y have larger pore size (approximately 10 A.) and differ as to the range of ratio of Al₂O₃ to SiO₂ as:
Type X--------------- Al₂O₃ /2.0-3.0 SiO₂
Type Y--------------- Al₂O₃/3.0-6.0 SiO₂
Type L and the other types listed have still higher ratios of SiO₂ to Al₂O₃.

The mole sieve catalysts employed in the present invention are the acid form mole sieves or exhibit acidic characteristics. The acid form of the mole sieves is commercially available, but also may be prepared by treating the mole sieves with acid to exchange Na for hydrogen. Another method to produce the acid form is to treat the mole sieve with decomposable cations (generally ammonium ions) to replace Na with the decomposable ions and thereafter to heat the mole sieve to decompose the cation leaving the acid form. Generally the Na form mole sieve is treated with ammonium hydroxide to remove the Na and thereafter the mole sieve is heated to a temperature of about 350°C to remove the ammonia. The removal of Na⁺ ions with NH⁺₄ is more easily carried out than with multivalent ions as described below and these catalysts are generally more active, but less stable to heat than the multivalent cation exchange forms. Mole sieves, which have had their alkali metal reduced to low levels by partial treatment with NH⁺₄ and partial multivalent metal cation exchange, possess increased activity and increased stability. In addition to mole sieves which are acidic according to the Bronsted Theory those mole sieves which exhibit acidic characteristics under the Lewis Theory, for example, calcium exchanged mole sieves are suitable for the present reaction. By exchanging the univalent cations (e.g.) Na⁺) with multivalent cations, strong ionic activity is imparted. The ratio of SiO₂:Al₂O₃, valence and radius of the cation and the extent of exchange all affect the catalyst activity. In general activity increases with (1) increased SiO₂ Al₂0₃ ratio, (2) decreased cation radius and an increase in cation valence. The effect of the replacing univalent ions (e.g. Na⁺ ) with bivalent (e.g. Ca⁺⁺) is much greater than replacing the bivalent ions with cations of greater valence.

The various types of mole sieves having reduced alkali metal content are characterized as the acid form molecular sieve and are all contemplated as useful in the present invention.

It would appear that the pore size within the crystal lattice may affect the selectivity. According to one theory of molecular sieve catalytic activity, zeolite catalysis occurs primarily inside the uniform crystal cavities, consequently zeolitic catalyst activity depends on the number of aluminum atoms in the crystal and thus on the chemical composition of the crystal. Moreover, these catalytic sites are fixed within the rigid structure of the crystal, so that access to site can be altered by altering the structure of the crystal.

The acid form mole sieves are generally produced and available as particles in the range of < 10 micron (powders) to 0,508 cm (0.2 inch) in diameter (beads).

In this form the mole sieves form too compact a bed and will not function adequately in a distillation, since there is a very large pressure drop through the bed and the free flow of internal reflux and rising vapor is impeded. Mole sieves in the shape of conventional distillation structures, such as rings, saddles, and the like may be used in the present invention. The particulate mole sieves may be employed by enclosing them in a porous container such as cloth, screen wire or polymeric mesh. The material used to make the container must be inert to the reactants and conditions in the reaction system. The cloth may be any material which meets this requirement such as cotton, fiber glass, polyester, nylon and the like. The screen wire may be aluminum, steel, stainless steel and the like. The polymer mesh may be nylon, teflon or the like. The mesh or threads per inch of the material used to make the container is such that the catalyst is retained therein and will not pass through the openings in the material. Particles of about 0.15 mm size or powders may be used and particles up to about 0,635 cm (1/4 inch) diameter may be employed in the containers.

The container employed to hold the catalyst particles may have any configuration, such as the pockets disclosed in the commonly assigned patents above,or the container may be a single cylinder, sphere, doughnut, cube, tube or the like.

Each container containing a solid catalytic material comprises a catalyst component. Each catalyst component is intimately associated with a spacing component which is comprised of at least 70 volume % open space up to about 95 volume % open space. This component may be rigid or resilient or a combination thereof. The combination of catalyst component and spacing component form the catalytic distillation structure. The total volume of open space for the catalytic distillation structure should be at least 10 volume % and preferably at least 20 volume % up to about 65 volume %. Thus desirably the spacing component or material should comprise about 30 volume % of the catalytic distillation structure, preferably about 30 volume % to 70 volume %. Resilient materials are preferred. One suitable such material is open mesh knitted stainless wire, known generally as demister wire or an expanded aluminum. Other resilient components may be similar open mesh knitted polymeric filaments of nylon, teflon and the like. Other materials such as highly open structures, foamed material,e.g. reticulated polyurethane foam (rigid or resilient), may be formed in place or applied around the catalyst component. In the case of larger catalyst components such as from about 0,635 cm (1/4 inch) to 1,27 cm (1/2) pellets, spheres, pills and the like each such larger component may be individually intimately associated with or surrounded by the spacing component as described above. It is not essential that the spacing component entirely cover the catalyst component; it is only necessary that the spacing component intimately associated with the catalyst component will act to space the various catalyst components away from one another as described above. Thus, the spacing component provides in effect a matrix of substantially open space in which the catalyst components are randomly but substantially evenly distributed.

A preferred catalytic distillation structure for use herein is made by placing the mole sieve or cation exchange resin particles into a plurality of pockets in a cloth belt, which is supported in the distillation column reactor by open mesh knitted stainless steel wire by twisting the two together in a helical form. This allows the requisite flows and prevents loss of catalysts. The cloth may be an material which is inert in the reaction. Cotton or linen are useful but fiber glass cloth or "Teflon" cloth are preferred.

In the following examples the catalyst packing 11 and 21 consisted of bags in the form of a fiber glass cloth belt approximately six inches wide with narrow pockets approximately 1,905 cm (3/4 inch) wide sewn across the belt. The pockets are spaced about 0,635 cm (1/4 inch) apart. These pockets are filled with the catalyst particles to form approximately cylindrical containers, and the open ends are then sewn closed to confine the particles. This belt is then twisted into a helical form to fit inside the column. Twisted in with the belt is also a strip of an open mesh knitted stainless steel wire, which serves to separate the mole sieve filled cloth pockets and provide a passage for vapor flow.

The wire mesh provides the support for the catalyst (belt) and provides some degree of vapor passage through the catalyst particles, which otherwise form a very compact bed which has a high pressure drop. Thus, the down flowing liquid is in intimate contact with the rising vapors in the column.

In commercial-scale operations, it is contemplated the catalyst packing would be made up of alternating layers of the acid cation exchange resin or mole sieve filled cloth belts similar to the ones described above, and a spacing material which could be of any convenient, suitable substance, such as a corrugated wire screen or wire cloth or a knitted wire mesh. The layers would be arranged vertically or horizontally. For simplicity of fabrication and for better distribution of vapor flow passages, a vertical orientation is preferred. The height of a section of this packing should be of any convenient dimension, from a few inches to several feet. For ease of assembly and installation, the packing would be made into sections of the desired shape and size, each section fastened together with circumferential bands of tie wires depending on its size and shape. A complete assembly in a column would consist of several sections, arranged in layers, with possibly the orientation of the catalyst-filled belts turned at right angles in successive layers to improve liquid and vapor flow distribution.

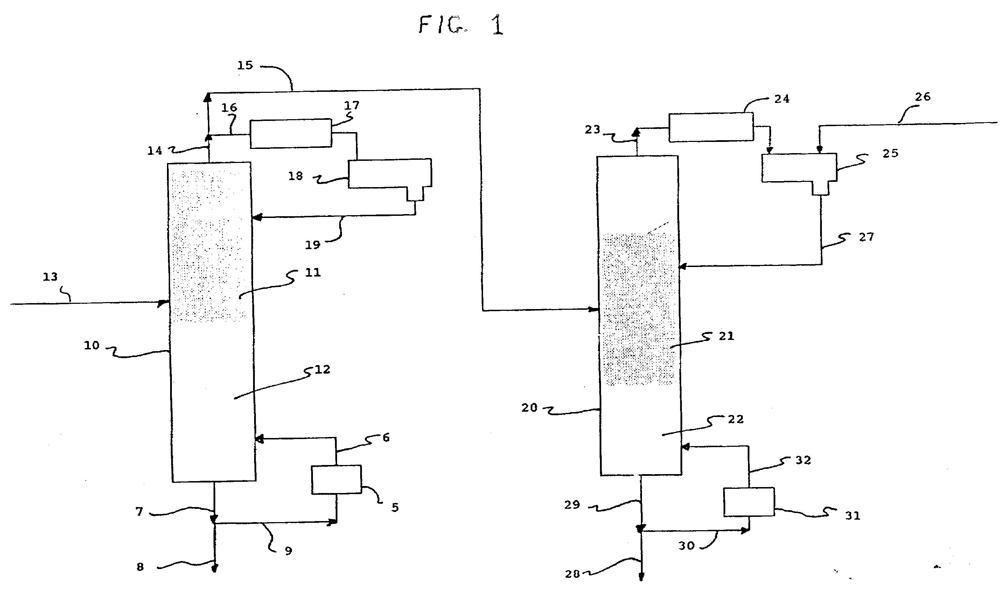

In one embodiment, shown in figure 1, acid cation exchange resin as prepared above is placed in a first distillation column reactor 10, the catalyst 11 occupying approximately the upper one third or one-half of the column. The acid cation exchange resin or mole sieve is prepared as above and loaded into a second distillation column reactor 20, the catalyst 21 occupying approximately the middle one-half of the second column. Inert packing is placed in the lower section of each column at 12 and 22 respectively.

A stream 13 containing a mixture of isobutene and n-butene and methanol is fed to the first column 10 at the lower portion of the catalyst packing 11 where the stream immediately contacts the acid cation exchange resin, reacting the iso-butene with the methanol to form methyl tertiary butyl ether. The first column is operated at a temperature and pressure such that the temperature in the reaction distillation zone 11 is at the boiling point of the methanol-butene mixture. As a result the ether, having a higher boiling point, will be distilled off the catalyst downward into inert packing 12 where any methanol or butenes carried therewith is allowed to boil back up into the reaction distillation zone. The substantially pure ether is taken out as bottoms, a portion of which may be heated in a reboiler 5 and returned to the column 10 via line 6 to supply heat. The unreacted n-butene is taken overhead via line 14, a portion being condensed in condenser 17 and collected in receiver 18 for reflux back to the distillation column reactor via line 19. The necessary conditions and preferred operations for the etherification of the isobutene with the methanol are given in U.S. Patent 4,336,407 which is hereby incorporated in its entirety by reference.

The portion of the unreacted n-butene not condensed is fed as a vapor via line 15 to the middle of catalyst bed 21 in second distillation column reactor 20. Benzene is fed as a liquid to the second distillation column reactor near the top of bed 21 via line 27 where it flows down the column and mixes with the vaporous n-butene. The mixture being in contact with the catalyst bed reacts to form butyl benzene. As in the first distillation column reactor, the second is operated at a temperature and pressure so as to be at the boiling point of the reacting mixture. The butyl benzene, having a higher boiling point than the mixture, is distilled off the catalyst down into the inert packing 22 where any of the reacting mixture is distilled back up to the reaction distillation zone 22. The butyl benzene is removed as bottoms via line 29 and some is recycled through reboiler 31 back to the column 20 to supply heat. Unreacted benzene is taken overhead via line 23 where it is condensed in condenser 24 and collected in receiver 25. As shown, make up benzene is added to receiver 25 also from which reflux to column 20 is returned to the distillation reaction zone 21 vis line 27.

In order to achieve high selectivity toward monosubstitution (which is a preferred aspect of the present invention), there is a large excess of the benzene to n-butene in the second reactor in the range of 2 to 100 moles of benzene per mole of n-butene, that is the net molar feed ratio of benzene to n-butene may be close to 1:1, although the system is operated so as to maintain a substantial molar excess of benzene to n-butene in the reaction zone. The adjustment is achieved by controlling the reflux of the benzene to the column. The alkylated product is the highest boiling material and is separated in the lower portion of the column usually as bottoms. Benzene is the second highest boiling component (excluding inserts) as noted above, however, by operating with a large excess of the organic aromatic compound, the major portion of the n-butene is reacted thereby reducing the separation and recovery problems.

In a second embodiment the n-butene is not completely reacted and condenser 24 is operated as a partial condenser to condense only the unreacted benzene and receiver 25 acts as a separator to remove the gaseous n-butene.

## Claims

1. A combined process for the production of methyl tertiary butyl ether and n-butyl benzene comprising in combination:
(a) feeding (1) a mixture containing isobutene and normal butene and (2) methanol to a first distillation column reactor into a first feed zone,
(b) concurrently in said first distillation column reactor
(1) contacting said mixture and said methanol with a first fixed bed acid cation exchange resin catalytic distillation structure in a first distillation reaction zone thereby catalytically reacting the isobutene with methanol to form methyl tertiary butyl ether and
(2) fractionating the resulting mixture and normal butene in said first fixed bed acid cation exchange resin catalytic distillation structure,
(c) withdrawing the ether from the first distillation column reactor at a point below said first feed zone,
(d) withdrawing the normal butene from the first distillation column reactor at a point above said first feed zone,
(e) Feeding said normal butene along with benzene to a second distillation column reactor into a second feed zone,
(f) concurrently in said second distillation column reactor
(1) contacting said normal butene and said benzene with a fixed bed acidic catalytic distillation structure in a second distillation reaction zone thereby catalytically reacting said normal butene with a portion of said benzene to form n-butyl benzene and
(2) fractionating the resultant mixture of unreacted benzene and n-butyl benzene in said second fixed bed acidic cation catalytic distillation structure,
(g) withdrawing the n-butyl benzene from said second distillation column reactor at a point below said second feed zone, and
(h) withdrawing said unreacted benzene from said second distillation column reactor at a point above said second feed zone.

2. The process according to claim 1 wherein said benzene is fed to said second feed zone in a molar excess over said n-butene thereby reacting substantially all of said n-butene with said benzene in said second distillation reaction zone.

3. The process according to claim 1 or 2 wherein a portion of said unreacted benzene is condensed and returned to said distillation column reactor to control the molar ratio of benzene to n-butene in said reaction distillation zone such that substantially all of said n-butene is reacted with said benzene in said second distillation reaction zone.

4. The process of claim 1 wherein only a portion of said n-butene reacts with said benzene and said unreacted n-butene is separated from said benzene.

5. The process according to claim 4 wherein said unreacted benzene is condensed and wherein said process further comprises returning a first portion of said condensed unreacted benzene to said second distillation column in inert packing as reflux and a second portion of said condensed unreacted benzene to said distillation reaction zone along with a portion of said unreacted n-butene to control the molar ratio of benzene to n-butene in said distillation reaction zone.

6. The process according to any one of Claims 1 to 5 wherein said first fixed bed catalytic distillation structure is cation exchange resin and said second fixed bed catalytic distillation structure is molecular sieve.

7. The process according to any one of Claims 1 to 5 wherein said first and second fixed bed catalytic distillation structures are acid cation exchange resin.

## Patentansprüche

1. Kombiniertes Verfahren zur Herstellung von Methyl-t-butylether und n-Butylbenzol, welches in seiner Kombination
(a) die Zuführung (1) einer Mischung mit einem Gehalt an Isobuten und n-Buten sowie (2) Methanol in eine erste Einleitungszone im Reaktor einer Destillationskolonne,
(b) gleichzeitig in besagtem Reaktor einer Destillationskolonne
(1) den Kontakt obigen Gemisches und Methanols mit einem ersten Aufbau aus saurem Festbett-Kationenaustauscherharz mit katalytischer Destillation in einer ersten Destillationsreaktionszone, wodurch das Isobuten mit dem Methanol zur Bildung von Methyl-t-butylether katalytisch zur Reaktion gebracht wird,
(2) die Fraktionierung des erhaltenen Gemisches und n-Buten in besagtem ersten Aufbau aus saurem Festbett-Kationenaustauscherharz mit katalytischer Destillation,
(c) das Entfernen des Ethers aus dem ersten Destillationskolonnenreaktor an einem Punkt unterhalb der ersten besagten Einspeisungszone,
(d) das Entfernen des n-Butens aus dem ersten Reaktor der Destillationskolonne an einem Punkt oberhalb der ersten besagten Einleitungszone
(e) die Zufuhr besagten n-Butens neben Benzol zu einem zweiten Destillationskolonnenreaktor in eine zweite Einleitungszone,
(f) gleichzeitig in besagtem zweiten Destillationskolonnenreaktor
(1) den Kontakt besagten n-Butens und Benzols mit einem sauren, katalytischen Festbett-Destillationsaufbau in einer zweiten Destillationsreaktionszone, wodurch das besagte n-Buten mit einer Teilmenge besagten Benzols zur Bildung von n-Butylbenzol katalytisch in Reaktion gebracht wird und
(2) das Fraktionieren des erhaltenen Gemisches aus unverbrauchtem Benzol und n-Butylbenzol in besagtem zweiten Aufbau aus saurem Festbett-Kationenaustauscherharz mit katalytischer Destillation,
(g) das Entfernen des n-Butylbenzols aus besagtem zweiten Destillationskolonnenreaktor an einem Punkt unterhalb besagter zweiter Einleitungszone und
(h) das Entfernen besagten nicht umgesetzten Benzols aus besagtem zweiten Destillationskolonnenreaktor an einem Punkt oberhalb besagter zweiter Einspeisungszone
umfaßt.

2. Verfahren nach Anspruch 1, worin das besagte Benzol der zweiten besagten Einleitungszone in molarem Überschuß im Vergleich zum besagten n-Buten zugeführt wird, wodurch im wesentlichen die Gesamtmenge besagten n-Butens mit besagtem Benzol in besagter zweiter Destillationsreaktionszone umgesetzt wird.

3. Verfahren nach Anspruch 1 oder 2, worin eine Teilmenge besagten nicht umgesetzten Benzols kondensiert und zu besagtem Destillationskolonnenreaktor zur Steuerung des molaren Verhältnisses von Benzol zu n-Buten in der Weise zurückgeführt wird, daß im wesentlichen die Gesamtmenge besagten n-Butens mit besagtem Benzol in besagter zweiter Destillationsreaktionszone umgesetzt wird.

4. Verfahren nach Anspruch 1, worin lediglich eine Teilmenge besagten n-Butens mit besagtem Benzol reagiert und besagtes nicht umgesetztes n-Buten von besagtem Benzol abgetrennt wird.

5. Verfahren nach Anspruch 4, worin besagtes nicht umgesetztes Benzol kondensiert wird und worin besagtes Verfahren ferner die Rückführung einer ersten Teilmenge besagten, kondensierten und nicht umgesetzten Benzols zur besagten zweiten Destillationskolonne in eine Inertpackung als Rückfluß und einer zweiten Teilmenge besagten, kondensierten und nicht umgesetzten Benzols zur besagten Destillationsreaktionszone neben einer Teilmenge besagten nicht umgesetzten n-Butens zur Steuerung des molaren Verhältnisses von Benzol zu n-Buten in besagter Destillationsreaktionszone umfaßt.

6. Verfahren nach einem der Ansprüche 1 bis 5, worin besagter erster saurer, katalytischer Festbett-Destillationsaufbau aus Kationenaustauscherharz besteht und besagter zweiter katalytischer Festbett-Destillationsaufbau ein Molekularsieb ist.

7. Verfahren nach einem der Ansprüche 1 bis 5, worin besagte erste und zweite saure, katalytische Festbett-Destillationsaufbauten aus saurem Kationenaustauscherharz bestehen.

## Revendications

1. Procédé combiné pour la production d'éther butylique tertiaire de méthyle et de n-butylbenzène, comprenant les étapes combinées consistant à :
(a) amener (1) un mélange contenant de l'isobutène et du butène normal et (2) du méthanol à un premier réacteur à colonne de distillation, dans une première zone d'amenée,
(b) parallèlement à cela, dans ledit premier réacteur à colonne de distillation :
(1) amener ledit mélange et ledit méthanol au contact d'une première structure de distillation catalytique à lit fixe de résine acide échangeuse de cations, dans une première zone de réaction de distillation, grâce à quoi on fait réagir catalytiquement l'isobutène avec le méthanol pour former l'éther butylique tertiaire de méthyle, et
(2) fractionner le mélange résultant et le butène normal dans ladite première structure de distillation catalytique à lit fixe de résine acide échangeuse de cations,
(c) retirer l'éther du premier réacteur à colonne de distillation en un point situé au-dessous de ladite première zone d'amenée,
(d) retirer le butène normal du premier réacteur à colonne de distillation en un point situé au-dessus de ladite première zone d'amenée,
(e) amener ledit butène normal avec du benzène à un deuxième réacteur à colonne de distillation, dans une deuxième zone d'amenée,
(f) parallèlement à cela, dans ledit deuxième réacteur à colonne de distillation :
(1) amener ledit butène normal et ledit benzène au contact d'une structure de distillation catalytique acide à lit fixe, dans une deuxième zone de réaction de distillation, grâce à quoi on fait réagir catalytiquement ledit butène normal avec une partie dudit benzène pour former du n-butylbenzène, et
(2) fractionner le mélange résultant de benzène n'ayant pas subi de réaction et de n-butylbenzène dans ladite deuxième structure de distillation catalytique acide de cations à lit fixe,
(g) retirer le n-butylbenzène dudit deuxième réacteur à colonne de distillation en un point situé au-dessous de ladite deuxième zone d'amenée, et
(h) retirer ledit benzène n'ayant pas subi de réaction dudit deuxième réacteur à colonne de distillation en un point situé au-dessus de ladite deuxième zone d'amenée.

2. Procédé selon la revendication 1, dans lequel ledit benzène est amené à la deuxième zone d'amenée avec un excès molaire par rapport audit n-butène, grâce à quoi on fait réagir pratiquement tout le n-butène avec le benzène dans ladite deuxième zone de réaction de distillation.

3. Procédé selon la revendication 1 ou 2, dans lequel une partie dudit benzène n'ayant pas subi de réaction est condensée et ramenée audit réacteur à colonne de distillation pour maîtriser le rapport molaire du benzène au n-butène dans ladite zone de réaction de distillation, de manière que pratiquement tout le n-butène réagisse avec ledit benzène dans ladite deuxième zone de réaction de distillation.

4. Procédé selon la revendication 1, dans lequel seulement une partie dudit n-butène réagit avec ledit benzène, et ledit n-butène n'ayant pas subi de réaction est séparé dudit benzène.

5. Procédé selon la revendication 4, dans lequel ledit benzène n'ayant pas subi de réaction est condensé, et ledit procédé comprend, en outre, le retour d'une première partie dudit benzène condensé n'ayant pas subi de réaction à une deuxième colonne de distillation, dans un emballage inerte, comme reflux, et d'une deuxième partie dudit benzène condensé n'ayant pas subi de réaction à ladite zone de réaction de distillation, conjointement avec une partie dudit n-butène n'ayant pas subi de réaction, pour maîtriser le rapport molaire du benzène au n-butène dans ladite zone de réaction de distillation.

6. Procédé selon l'une quelconque des revendications 1 à 5, dans lequel ladite première structure de distillation catalytique à lit fixe est une résine échangeuse de cations, et ladite deuxième structure de distillation catalytique à lit fixe est un tamis moléculaire.

7. Procédé selon l'une quelconque des revendications 1 à 5, dans lequel lesdites première et deuxième structures de distillation catalytique à lit fixe sont une résine acide échangeuse de cations.
